# EUROPEAN PATENT SPECIFICATION

(11) **EP 0 599 590 B1**
(45) Date of publication and mention of the grant of the patent: **09.02.2000**
(21) Application number: 93309307.2
(22) Date of filing: 22.11.1993
(51) Int. Cl.: A61B 8/00, G10K 11/00

(54) **Intraoperative ultrasound probe**
Intraoperativer Ultraschallfühler
Sonde à ultra-son intra-opératoire

(30) Priority: 23.11.1992 GB 9224585
(43) Date of publication of application: 01.06.1994
(60) Divisional application: 99200175.0
(73) Proprietor: ADVANCED TECHNOLOGY LABORATORIES, INC., Bothell, Washington 98041 (US)
(72) Inventor: Nordgren, Timothy F., Bothell, Washington 98021 (US); Imling, Deborah K., Washington 98008 (US); Ungari, Joseph L., Everett, Washington 98206 (US); Killam, Donald Gilbert, Woodinville, Washington 98072 (US); McKeighen, Ronald E., Woodinville, Washington 98072 (US)
(74) Representative: Lottin, Claudine

(56) References cited:
- EP-A- 0 093 277
- EP-A- 0 213 859
- EP-A- 0 366 405
- DE-A- 3 810 289
- US-A- 4 494 548

## Description

This invention relates to ultrasonic diagnostic probes or scanheads and, in particular, to ultrasound probes which are uniquely suited for imaging and diagnosing organs of the body and arterial vessels during surgery.

Ultrasonic diagnostic imaging probes, which generally find application during noninvasive procedures, can also be used during surgical procedures. One such procedure where ultrasonic imaging probes have particular utility is during vascular surgery. During vascular surgery, ultrasonic imaging probes can be utilized to image and diagnose the interior of carotid arteries. Another procedure where ultrasonic imaging probes have utility is during transplant surgery where, for example, the ultrasonic imaging probe can be used to verify successful attachment and function of renal arteries.

Surgical ultrasound probes are preferably small and as easily to manipulate as surgical instruments. Such probes should be capable of imaging the blood flow of a vessel which is in immediate contact with the probe. This necessitates focal properties of the probe which enable it to produce a focused image of tissue and structure which is in immediate contact with the probe. Reliability is enhanced by the provision of an all electronic probe with no mechanically moving parts. When using surgical probes it is important that the hand of the physician not obscure the surgical site being examined. Such probes must be adapted for use in a sterile surgical field, must pose no electrical hazard to the patient, and must be easy to clean and sterilize, e.g., should be smoothly and securely integrated and closed with no gaps or openings. Furthermore, such probes should be capable of use around organs and under skin which has not been incised during the surgical procedure.

EP-A-0 093 277 describes an intraoperative ultrasonic transducer probe as defined in the preamble to claim 1.

In accordance with the present invention there is provided an intraoperative ultrasonic transducer probe as defined in claim 1. The transducer is preferably a piezoelectric, electronically steered and focused multielement transducer with no moving parts. The transducer end of the probe is preferably contained within an encapsulating boot and lens material, which provides reliable electronic insulating properties and is easy to clean and sterilize. The encapsulating boot also performs the function of a standoff, with the multielement transducer being capable of focusing at the tissue which is in immediate contact with the boot. The angulation of the handle permits the physician to grasp and manipulate the probe without blocking the physician's view of the surgical site. The offset attachment of the transducer end and shaft of the probe enables the physician to insert the distal tip of the transducer end under skin which has not been incised to examine tissues and arterial vessels peripheral to the point of incision.

In the drawings:
FIGURE 1 illustrates the outline of an intraoperative ultrasound probe of the present invention;
FIGURE 2 illustrates a side view of the interior of the intraoperative ultrasound probe of FIGURE 1;
FIGURES 2a-2d illustrate various bottom, end and cross-sectional views of the probe of FIGURE 2;
FIGURE 3 is a partially cutaway view of the distal end of the probe of FIGURE 2;
FIGURE 4, 5, and 6 illustrate the manner in which the cable is attached to an intraoperative ultrasound probe of the present invention;
FIGURES 7a-7e illustrate the construction of a preferred termination assembly for connecting a multifilament coaxial cable to the intraoperative ultrasound probe of the present invention; and
FIGURE 8 illustrates the fully assembled termination assembly of FIGURES 7a-7e.

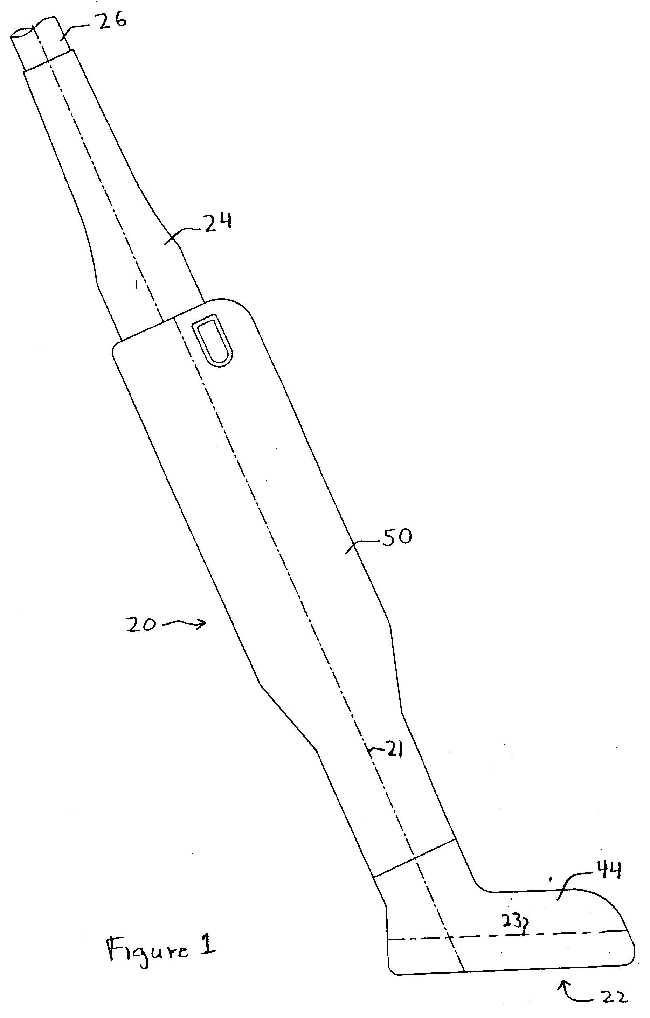

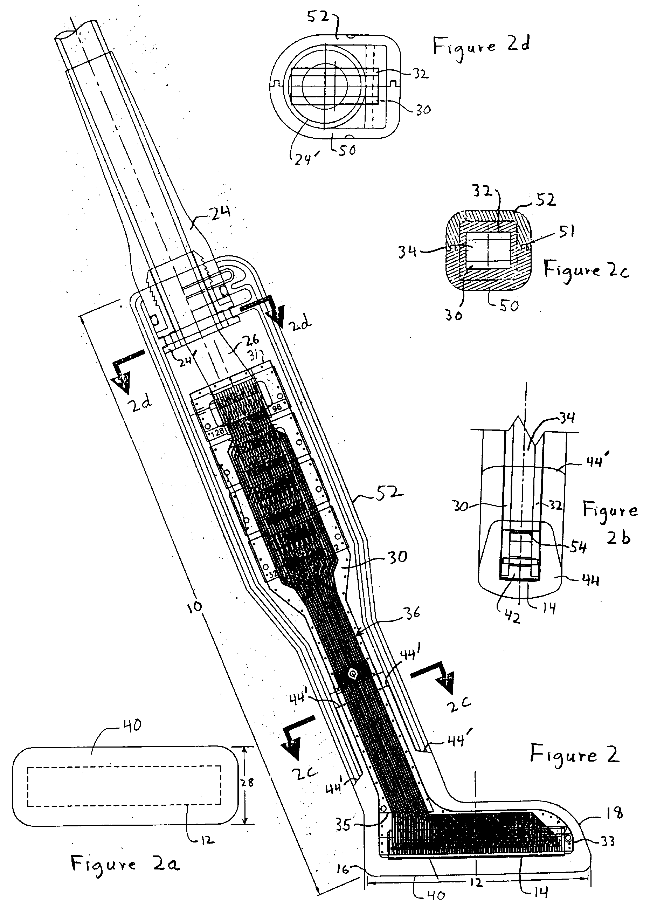

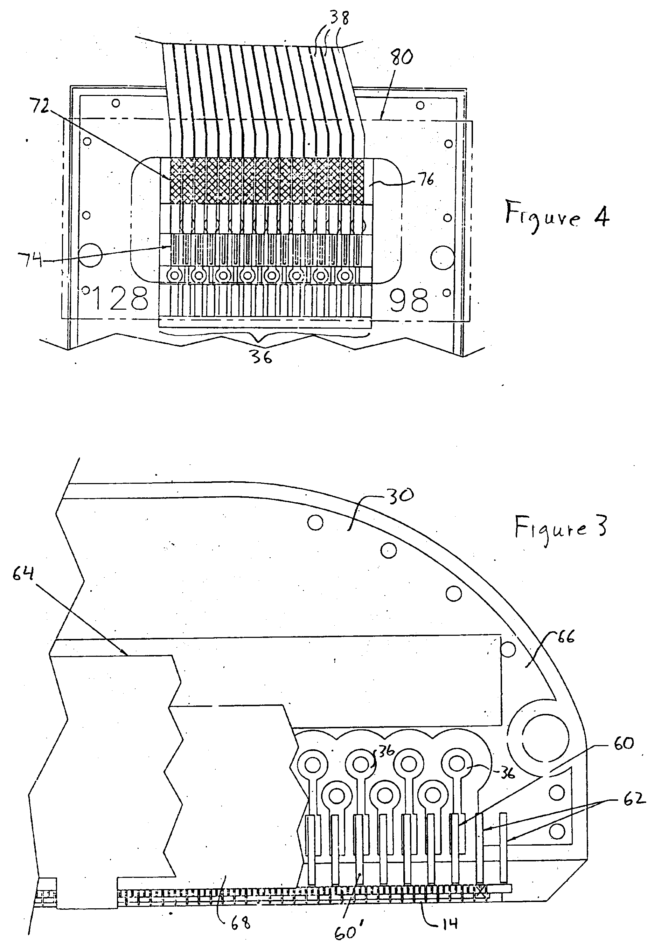

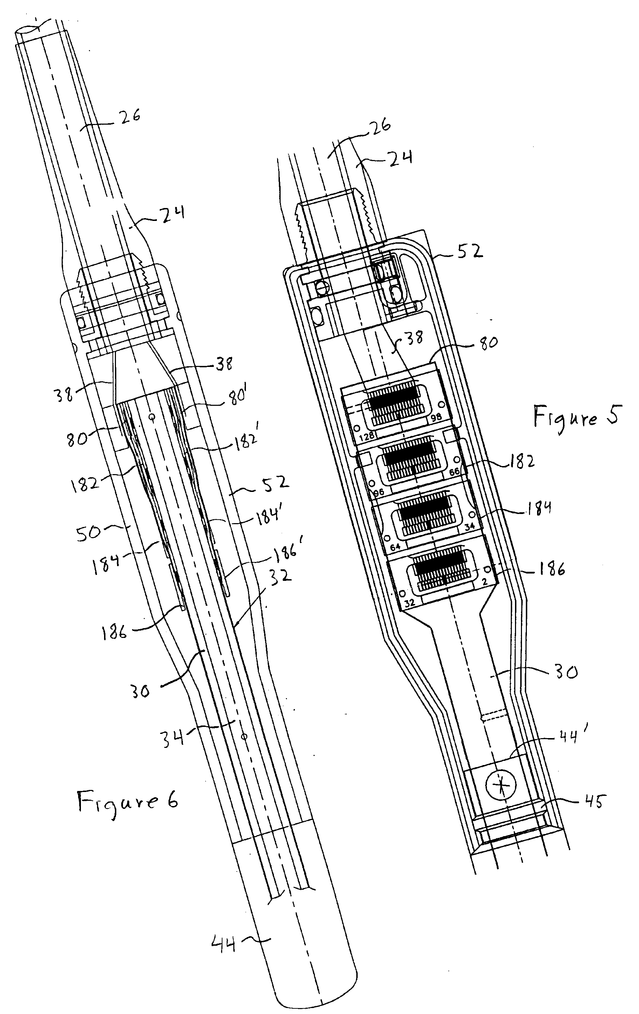

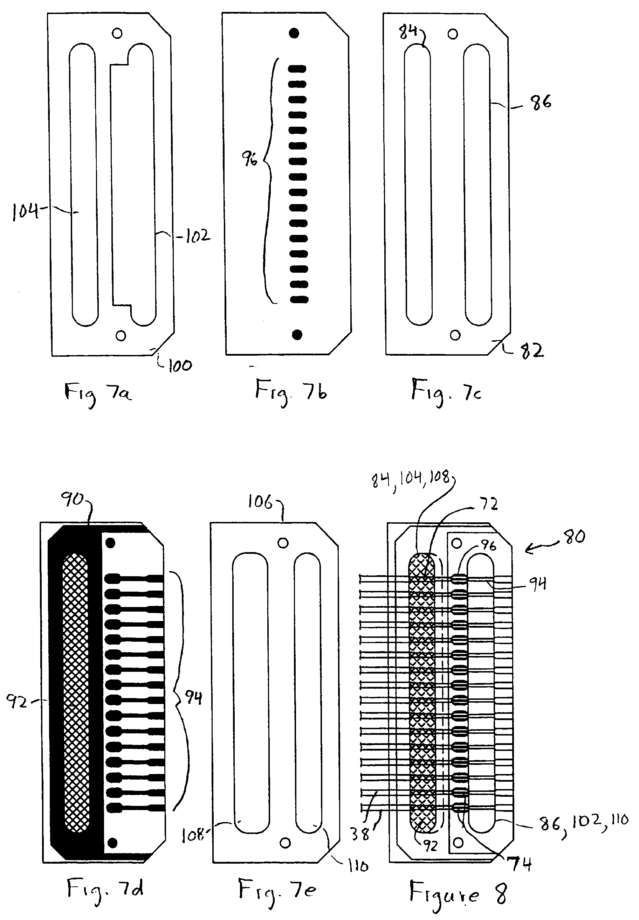

Referring first to FIGURES 1 and 2, an intraoperative ultrasound probe constructed in accordance with the principles of the present invention and specifically designed for vascular surgical applications is shown. The probe is approximately L-shaped, resembling a tiny model of a leg and foot. The probe comprises a distal transducer section 22 resembling a boot on the "foot" and a handle section 20 resembling the "leg", with a strain relief 24 and multifilament cable 26 exiting the proximal end of the handle section 20. The cable leads to a connector (not shown) suitable for connecting the probe to an ultrasonic diagnostic system which drives the transducer of the probe and receives ultrasonic echo signals from the transducer in the conventional manner. A case with halves 50 and 52 form the exterior of the handle section 20 and the transducer section boot 44 is formed of molded rubber. The major axis 23 of the transducer section 22 is inclined at an angle of 112° with respect to the major axis 21 of the handle section. The length 10 of the handle section 20 from its proximal end to the heel 16 of the transducer section 22 is approximately 95 mm. The length 12 of the transducer section from heel 16 to its toe 18 is approximately 34.5 mm and the width 28 of the transducer section (FIGURE 2a) is approximately 12 mm.

Referring now to FIGURES 2 and 2a-2d, two matched printed circuit boards 30 and 32 are located inside the case 50, 52 and boot 44, each board having the inclined L-shape of the probe. Each printed circuit board extends from a top end 31 to a bottom distal end 33. The printed circuit boards are mounted in parallel, separated as shown in FIGURES 2b-2d by an insulating spacer 34, extending from the top 31 of the printed circuit boards to a lower terminating edge 35. Each printed circuit board contains a plurality of conductive traces 36, which make electrical connections between the transducer crystal 14 and the upper surfaces of the boards where connections are made to wires 38 of the multifilament cable 26.

The transducer crystal 14 is mounted perpendicular to the lower edges of the printed circuit boards 30 and 32 as shown in FIGURES 2, 2a, and 2b. The crystal 14 has an overall length of approximately 27 mm and a width of approximately 4.6 mm. The crystal 14 is diced into an array of 128 finely pitched elements for the performance of B-scan and color flow Doppler imaging. The finely pitched elements transmit and receive over an operating frequency range of 5-10 MHz, giving the transducer the ability to visualize and diagnose a vessel which is immediately in contact with the footprint 40 of the probe. The footprint of the probe is shown in the bottom view of FIGURE 2a, with the outline 12 of the transducer crystal indicated by a dashed line box.

The transducer crystal 14 is plated on its upper and lower surfaces with layers of conductive foil, one of which is cut by the dicing process. A plurality of metallic TAB leads are bonded at one end to the diced conductive foil with the other end of each TAB lead being bonded to a conductive trace 36 at the edges of the printed circuit boards 30 and 32. TAB lead connections are made to both the ground (undiced) and signal (diced) side of each piezoelectric element of the crystal and thence to the printed circuit boards 30 and 32.

Turning to FIGURE 3, the manner in which electrical connections are made between the elements of the transducer crystal 14 and the conductive traces 36 of the printed circuit boards 30 and 32 is shown in enlarged detail. TAB leads 60, 60' are soldered at one end to the conductive foil strips on the inner surface of the transducer crystal 14. These strips have been formed, as mentioned above, by the dicing process. Two TAB leads 62 are connected to foil strips of inactive elements at the end of the transducer array. The other ends of the TAB leads 60 and 60' are soldered to conductive traces 36 on the printed circuit board 30. The ends of TAB leads 62 are soldered to the ground plane 66 of the printed circuit board.

A ground foil 64 folds over the outer foil surface of the transducer crystal and is attached to that foil surface to ground it. The ground foil 64 is also electrically connected to the ground plane 66 of the printed circuit board. A sheet 68 of nonconductive material insulates the conductive traces 36 from the ground foil 64.

It will also be noted in FIGURE 3 that TAB leads are only connected to alternating transducer elements. In this preferred embodiment, electrical connection is made to odd numbered elements from printed circuit board 32, and to even numbered elements from printed circuit board 30.

Returning to FIGURE 2 and 2a-2e, the volume 42 behind the transducer crystal and below the terminating edge 35 of the spacer 34, and between the printed circuit boards, is filled with a loaded epoxy backing material to a depth as indicated by the jagged upper surface 54 of the backing material in FIGURE 2b. The backing material attenuates acoustic waves emanating from the rear of the crystal. The entire transducer section is then encased in an RTV rubber compound by a molding process which forms the boot 44. As FIGURES 2 and 2b show, the RTV rubber casing 44 is approximately 2.5 mm thick below the transducer 14. This gives the probe an inherent standoff distance between the crystal and the vascular structure of the body that is in contact with the footprint 40 of the probe. The standoff distance allows vessels in contact with the footprint to be clearly imaged, as the standoff distance enables the return of ultrasonic echoes from the contacted vessel to return to a plurality of transducer elements for focusing in flow and image processing.

The RTV rubber casing 44 extends to edges 44' along the handle section of the probe and inside the case halves 50 and 52. If desired, the molding process can also form an integral liquid tight gasket 45 in the rubber where the lower portion of the case closes around the boot (see FIGURE 5). The encapsulating boot thus completely seals the transducer end of the probe, fully insulating the patient from the electrical connections to the transducer crystal. The encapsulating boot also renders the probe easy to clean and sterilize between uses, further enhancing patient safety and medical efficiency.

The handle section 20 above the edges 44' of the RTV rubber casing 44 comprises two hollow clamshell case halves 50 and 52 formed of a rigid polysulfone plastic. The case 50, 52 encloses the upper extension of the printed circuit boards 30 and 32 where connections are made to to the wires 38 of the cable 26. One technique for attaching the wires 38 of the cable 26 to the printed circuit boards 30 and 32 is shown in FIGURE 4, 5, and 6.

The preferred cable is comprised of 128 individual coaxial conductors. In FIGURE 4 reference numeral 38 designates the outer sheaths of several of these coaxial conductors. The outer sheaths terminate to expose the braided ground shields 72 of the coax, and these ground shields are soldered to a grounding pad 76 on the printed circuit board. The ground shields terminate and following a short length of inside insulating sheath the center conductors 74 of the coax are exposed. These center conductors are soldered to individual conductive signal traces 36 on the printed circuit board.

As FIGURE 5 shows, the coax wires are connected to the boards in groups of sixteen as denoted by terminating assemblies 80, 182, 184, and 186. Each terminating assembly terminates at a different distance along the cable so that the wires of each terminating assembly overlay the previous assemblies when attached to their positions on the printed circuit boards. This overlap is shown in FIGURE 6. In correspondence with the electrical connections to the transducer elements, odd numbered wires are connected to one of the printed circuit boards, and even numbered wires are connected to the other board. For ease of illustration of the termination assemblies, the overlapping wires have not been shown in FIGURE 5 but are illustrated in the profile view of FIGURE 6.

Preferably the cables are preassembled with the conductors of the coaxial wires soldered to terminating assemblies. The terminating assemblies are then easily soldered in position on the printed circuit boards 30 and 32. A preferred terminating assembly is shown in FIGURES 7a-7e and 8. Each terminating assembly is a multilayered panel of copper foil patterns and insulating sheets made of a flexible, insulating polyimide material. The terminating assembly includes a center polyimide sheet 82 as shown in FIGURE 7c. Photoetched on one side of the center sheet 82 is a line 96 of sixteen foil pads. Photoetched on the other side of the center sheet is a ground plane 90 with a central screen-like foil pattern 92, and sixteen bridging lines 94. Electrical through hole connections known as vias connect each pad 96 to a bridging line 94 on the other side of the center sheet 82. Apertures 84 and 86 are then formed in the center sheet 82, resulting in bridging lines 94 bridging the aperture 86 and the screen-like pattern 92 extending across aperture 84.

The assembly is completed by laminating an upper polyimide sheet 100 to the top of the center sheet, with aperture 102 exposing pads 96 and bridging lines 94, and aperture 104 exposing the copper screen 92. A lower polyimide sheet 106 with aligning apertures is laminated to the other side of the center sheet.

Each terminating assembly is attached to sixteen coaxial wires of the cable 26 as shown in FIGURE 8. The braided ground shields 72 are soldered to the screen-like foil pattern 92. During this process it is intended that solder will flow through the holes in the screen to the back side of the screen. The center coaxial conductors 74 are soldered to the individual foil pads 96 through aperture 102. Electrical connection is formed from the conductors 74 to the bridging lines 94 through the interconnecting vias.

Attachment of the cable to printed circuit boards 30 and 32 is now quick and easy. The screen-like foil pattern is positioned over the grounding pad 76 on the printed circuit board with the bridging lines 94 aligned with their mating conductive traces 36 on the board. In one operation the solder which flowed through to the back of the screen 92 is heated to attach the ground shields and screen to the pad 76. If an insufficient amount of solder flowed to the back side of the screen during the prior assembly step, this soldering step may be augmented by providing additional solder to the ground shields so that the shields and screen a securely soldered to the pad 76. A second operation simultaneously contacts bridging lines 94 through aperture 102 and solders the lines 94 to the underlying conductive traces 36. The soldering of the bridging lines should not loosen the conductors 74 from pads 96 due to the low thermal conductivity of the electrically interconnecting vias. In this manner the eight terminating assemblies 80, 80', 182-196 and 182'-186' are quickly attached to the printed circuit boards of the probe.

After all of these electrical connections have been made, the case 50, 52 is closed around the RTV rubber casing 44 at the distal end of the case and a flange 24' of the strain relief 24 at the proximal end of the case and sealed with an RTV adhesive to form a liquidtight enclosure, thereby enabling the probe to be safely and reliably used in a surgical field and thereafter sterilized.

As FIGURES 2c and 2d show, at the wider top of the handle the case is rounded in the back with squared corners in the front (FIGURE 2d), and makes a transition to its narrower, lower dimension where it is approximately square in cross-section (FIGURE 2c). This shape enables the surgeon to hold and handle the probe in the manner of a surgical instrument, with the rounded back resting between the thumb and forefinger and the squared corners affording a secure grip for the fingers.

The inclined L-shape of the probe enables the surgeon to grasp the handle section 20 and manipulate the probe without the hand obscuring the surgical site and vessel being contacted by the probe footprint 40. The transducer section 22 can be moved along a vessel to image the vessel even beyond the extent of an incision, because the toe 18 of the transducer section can be inserted under the skin at the end of the incision. This enables the surgeon to view and diagnose flow conditions in the vessel beyond the incision site.

## Claims

1. An intraoperative ultrasonic transducer probe comprising:
a patient-contacting section containing a multielement ultrasonic transducer (14) opposing a patient-contacting surface of the probe, said patient-contacting section having a longitudinal axis aligned with the longitudinal axis (23) of said ultrasonic transducer (14); and
a handle section (20) suitable for being held by a physician during use of the probe, said handle section having a longitudinal axis (21) and including means (26) for connecting said probe to an ultrasonic diagnostic system, said handle section (20) being attached to said patient-contacting section at one end of said handle section (20) and at a point on said patient-contacting section which is offset from the center of said patient-contacting section; characterised in that said longitudinal axis (21) of said handle section (20) intersects said patient-contacting section at said offset point; and in that the angle between said longitudinal axis of said patient-contacting section and said longitudinal axis (21) of said handle section (20) is an obtuse angle.

2. The intraoperative ultrasonic transducer probe of Claim 1, wherein the exterior of said patient-contacting section comprises an insulating material which encapsulates said ultrasonic transducer (14).

3. The intraoperative ultrasonic transducer probe of Claim 2, wherein said insulating material further comprises a spatial offset between the surface of said multielement transducer (14) and the patient-contacting surface of said probe, wherein said multielement transducer (14) is capable of being focused at the surface of material in contact with said patient-contacting surface.

4. The intraoperative ultrasonic transducer probe of claim 2 or claim 3, wherein said insulating material further comprises a liquidtight, sterilizable encapsulation of said multielement transducer (14).

5. The intraoperative ultrasonic transducer probe of any one of claims 2 to 4, wherein said insulating material further comprises a moldable, rubber-like material.

6. The intraoperative ultrasonic transducer probe of any preceding claim, further comprising:
an angled member (30, 32, 34) angled at said obtuse angle and extending into said patient-contacting and handle (20) sections and carrying at least one printed circuit comprising a plurality of conductive traces (36) connected in the patient-contacting section to elements of said ultrasonic transducer (14) and connected in the handle section (20) to said connecting means (26).

7. The intraoperative ultrasonic transducer probe of Claim 6, wherein said angled member (30, 32, 34) is located in a plane which is parallel to said longitudinal axis of said patient-contacting section, said longitudinal axis (23) of said ultrasonic transducer (14), and said longitudinal axis (21) of said handle section (20).

8. The intraoperative ultrasonic transducer probe of Claim 6 or Claim 7, wherein said angled member (30, 32, 34) is a rigid, planar member.

9. The intraoperative ultrasonic transducer probe of any one of Claims 6 to 8, wherein said angled member (30, 32, 34) comprises a printed circuit board (30) having said plurality of conductive traces (36) formed thereon.

10. The intraoperative ultrasonic transducer probe of Claim 9, wherein said angled member further comprises a second printed circuit board (32) located in parallel with said first named printed circuit board (30) and having a further plurality of conductive traces (36) formed thereon for coupling elements of said multielement transducer (14) to said connecting means (26).

## Patentansprüche

1. Intraoperativer Ultraschallfühler mit:
einem Abschnitt, der in Berührung mit dem Patienten kommt und einen Multielement-Ultraschallmeßwandler (14) enthält, der gegenüber einer Oberfläche des Fühlers in Berührung mit dem Patienten liegt, wobei die Längsachse des genannten Patientenkontakt-Abschnitts auf die Längsachse (23) des genannten Ultraschallmeßwandlers (14) ausgerichtet ist; und mit
einem Handgriffabschnitt (20), den ein Arzt während der Benutzung des Fühlers in der Hand halten kann, wobei der Handgriffabschnitt eine Längsachse (21) besitzt und Mittel (26) enthält, um den genannten Fühler mit einem Ultraschalldiagnosesystem zu verbinden, wobei der Handgriffabschnitt (20) an einem Ende mit dem genannten Patientenkontakt-Abschnitt verbunden ist, und zwar an einem Punkt auf dem genannten Patientenkontakt-Abschnitt, der in Bezug auf die Mitte des genannten Patientenkontakt-Abschnitts versetzt ist; dadurch gekennzeichnet, daß die genannte Längsachse (21) des genannten Handgriffabschnitts (20) den Patientenkontakt-Abschnitt bei dem genannten Versatzpunkt schneidet; und daß der Winkel zwischen der genannten Längsachse des genannten Patientenkontakt-Abschnitts und der genannten Längsachse (21) des genannten Handgriffabschnitts (20) ein stumpfer Winkel ist.

2. Intraoperativer Ultraschallfühler nach Anspruch 1, wobei die Außenseite des genannten Patientenkontakt-Abschnitts aus einem Isoliermaterial besteht, das den genannten Ultraschallmeßwandler (14) umschließt.

3. Intraoperativer Ultraschallfühler nach Anspruch 2, wobei das genannte Isoliermaterial einen räumlichen Versatz zwischen der Oberfläche des genannten Multielement-Meßwandlers (14) und der Patientenkontakt-Oberfläche des genannten Fühlers umfaßt, wobei der genannte Multielement-Meßwandler (14) auf die Oberfläche des Materials in Kontakt mit der genannten Patientenkontakt-Oberfläche fokussiert werden kann.

4. Intraoperativer Ultraschallfühler nach Anspruch 2 oder 3, wobei das genannte Isoliermaterial außerdem eine flüssigkeitsdichte, sterilisierbare Verkapselung des genannten Multielement-Meßwandlers (14) umfaßt.

5. Intraoperativer Ultraschallfühler nach einem der Ansprüche 2 bis 4, wobei das genannte Isoliermaterial weiterhin ein formbares, gummiähnliches Material umfaßt.

6. Intraoperativer Ultraschallfühler nach einem der vorhergehenden Ansprüche, der weiterhin folgendes umfaßt:
ein gewinkeltes Element (30, 32, 34), das in einem stumpfen Winkel gewinkelt ist und sich in den genannten Patientenkontakt-Abschnitt und den genannten Handgriffabschnitt hinein erstreckt und mit mindestens einer gedruckten Schaltung versehen ist, die eine Vielzahl von Leiterbahnen (36) umfaßt, welche in dem Patientenkontakt-Abschnitt mit Elementen des genannten Ultraschallmeßwandlers (14) verbunden sind und in dem Handgriffabschnitt (20) mit den genannten Verbindungsmitteln (26) verbunden sind.

7. Intraoperativer Ultraschallfühler nach Anspruch 6, wobei das genannte gewinkelte Element (30, 32, 34) in einer Ebene angeordnet ist, die parallel zu der genannten Längsachse des genannten Patientenkontakt-Abschnitts, zu der genannten Längsachse (23). des genannten Ultraschallmeßwandlers (14) und zu der genannten Längsachse (21) des genannten Handgriffabschnitts (20) verläuft.

8. Intraoperativer Ultraschallfühler nach Anspruch 6 oder 7, wobei das genannte gewinkelte Element (30, 32, 34) ein starres, planares Element ist.

9. Intraoperativer Ultraschallfühler nach einem der Ansprüche 6 bis 8, wobei das genannte gewinkelte Element (30, 32, 34) eine Leiterplatte (30) mit einer genannten Vielzahl von Leiterbahnen (36) darauf umfasst.

10. Intraoperativer Ultraschallfühler nach Anspruch 9, wobei das genannte gewinkelte Element außerdem eine zweite Leiterplatte (32) parallel zu der ersten genannten Leiterplatte (30) umfasst, und mit einer weiteren Vielzahl von Leiterbahnen (36) darauf, um Elemente des genannten Multielement-Meßwandlers (14) mit den genannten Verbindungsmitteln (26) zu verbinden.

## Revendications

1. Sonde à transducteur à ultrasons intra-opératoire, comprenant :
une section en contact avec le patient contenant un transducteur à ultrasons multiélément (14) opposé à une surface de la sonde en contact avec le patient, ladite section en contact avec le patient ayant un axe longitudinal aligné avec l'axe longitudinal (23) dudit transducteur à ultrasons (14), et
une section de poignée (20) destinée à être tenue par un médecin au cours de l'utilisation de la sonde, ladite section de poignée ayant un axe longitudinal (21) et comprenant un moyen (26) pour connecter ladite sonde à un système diagnostique à ultrasons, ladite section de poignée (20) étant fixée à ladite section en contact avec le patient à une extrémité de ladite section de poignée (20) et en un point sur ladite section en contact avec le patient qui est décalé du centre de ladite section en contact avec le patient, caractérisé en ce que ledit axe longitudinal (21) de ladite section de poignée (20) coupe ladite section en contact avec le patient au niveau dudit point décalé; et en ce que l'angle entre ledit axe longitudinal de ladite section en contact avec le patient et ledit axe longitudinal (21) de la section de poignée (20) est un angle obtus.

2. Sonde à transducteur à ultrasons intra-opératoire selon la revendication 1, dans laquelle l'extérieur de ladite section en contact avec le patient comprend un matériau isolant qui encapsule ledit transducteur à ultrasons (14).

3. Sonde à transducteur à ultrasons intra-opératoire selon la revendication 2, dans laquelle ledit matériau isolant comprend par ailleurs un décalage spatial entre la surface dudit transducteur multiélément (14) et la surface en contact avec le patient de ladite sonde, dans laquelle ledit transducteur multiélément (14) est à même d'être focalisé à la surface du matériau en contact avec ladite surface en contact avec le patient.

4. Sonde à transducteur à ultrasons intra-opératoire selon la revendication 2 ou 3, dans laquelle ledit matériau isolant comprend par ailleurs une encapsulation étanche aux liquides stérilisable dudit transducteur multiélément (14).

5. Sonde à transducteur à ultrasons intra-opératoire selon l'une quelconque des revendications 2 à 4, dans laquelle ledit matériau isolant comprend par ailleurs un matériau de type caoutchouc moulable.

6. Sonde à transducteur à ultrasons intra-opératoire selon l'une quelconque des revendications précédentes, comprenant par ailleurs :
un élément angulaire (30, 32, 34) qui forme ledit angle obtus et qui s'étend dans ladite section en contact avec le patient et dans ladite section de poignée (20) et portant au moins un circuit imprimé comprenant une pluralité de traces conductrices (36) connectées, dans la section en contact avec le patient, à des éléments dudit transducteur à ultrasons (14) et connectées, dans la section de poignée (20), audit moyen de connexion (26).

7. Sonde à transducteur à ultrasons intra-opératoire selon la revendication 6, dans laquelle ledit élément angulaire (30, 32, 34) est situé dans un plan qui est parallèle audit axe longitudinal de ladite section en contact avec le patient, audit axe longitudinal (23) dudit transducteur à ultrasons (14) et audit axe longitudinal 21) de ladite section de poignée (20).

8. Sonde à transducteur à ultrasons intra-opératoire selon la revendication 6 ou 7, dans laquelle ledit élément angulaire (30, 32, 34) est un élément plan rigide.

9. Sonde à transducteur à ultrasons intra-opératoire selon l'une quelconque des revendications 6 à 8, dans laquelle ledit élément angulaire (30, 32, 34) comprend une carte à circuit imprimé (30) sur laquelle est formée ladite pluralité de traces conductrices (36)

10. Sonde à transducteur à ultrasons intra-opératoire selon la revendication 9, dans laquelle ledit élément angulaire comprend par ailleurs une seconde carte à circuit imprimé (32) disposée en parallèle avec ladite carte à circuit imprimé (30) mentionnée en premier lieu et portant une autre pluralité de traces conductrices (36) pour coupler les éléments dudit transducteur multiélément (14) avec ledit moyen de connexion (26).
